# EUROPÄISCHE PATENTANMELDUNG

(11) **EP 2 803 326 A1**
(43) Veröffentlichungstag der Anmeldung: **19.11.2014**
(21) Anmeldenummer: 14165907.8
(22) Anmeldetag: 24.04.2014
(51) Int. Cl.: A61B 17/29, A61B 17/00

(54) **Biegbahrer Rohrschaft**

(30) Priorität: 13.05.2013 DE 102013208728
(71) Anmelder: AESCULAP AG, 78532 Tuttlingen (DE)
(72) Erfinder: Kupferschmid, Bernhard, 78576 Emmingen-Liptingen (DE)
(74) Vertreter: Koller, Tobias Kilian

(57) **Zusammenfassung**

Die vorliegende Anmeldung offenbart einen Rohrschaft für ein Rohrschaftinstrument mit einem hohlen Schaftbauteil (20), einem in dem hohlen Schaftbauteil (20) angeordneten Betätigungsstab (30), und Funktionselementen, die an den distalen Enden des Schaftbauteils (20) und/oder des Betätigungsstabs (30) angebracht sind. Der Betätigungsstab (30) ist relativ zu dem hohlen Schaftbauteil (20) in axialer Richtung verschiebbar, um dadurch die distalen Abschnitte der Funktionselemente aufeinander zu, aneinander vorbei und/oder voneinander weg zu bewegen. Der Betätigungsstab (30) weist wenigstens einen Bereich (32, 33) auf, in dem sich flexible Abschnitte (34, 34', 34") und Stützabschnitte (35, 35', 35") abwechseln und in dem der Betätigungsstab (30) zumindest in einer Querrichtung einen deutlich geringeren Biegewiderstand aufweist als außerhalb dieses mindestens einen Bereichs (32, 33). Zudem weist das hohle Schaftbauteil (20) mindestens einen Bereich (21) auf, in dem die Wand des Schaftbauteils (20) Aussparungen (22) aufweist und das Schaftbauteil (20) zumindest in einer Querrichtung einen deutlich geringeren Biegewiderstand aufweist als außerhalb dieses mindestens einen Bereichs (21). Die mindestens einen Bereiche (21, 32, 33) des Betätigungsstabs (20) und des Schaftbauteils überlagern sich in Längsrichtung des Rohrschafts zumindest teilweise. Eine reibungsmindernde Schicht ist an dem wenigstens einen Bereich (32, 33) des Betätigungsstabs vorgesehen, welche die Reibung des Betätigungsstabes (30) an der Innenwand des Schaftbauteils (20) verringert. Bevorzugt ist die reibungsmindernde Schicht bevorzugt durch einen Schrumpfschlauch gebildet.

## Beschreibung

Die vorliegende Erfindung betrifft einen Rohrschaft für ein Rohrschaftinstrument und insbesondere einen Rohrschaft, der durch den Operateur händisch in eine passende Form gebogen werden kann.

Im Stand der Technik sind zahlreiche Rohrschaftinstrumente und daher auch zahlreiche Rohrschäfte bekannt. In der europäischen Patentanmeldung EP 0 577 423 A2 ist beispielsweise ein klassisches Rohrschaftinstrument gezeigt bei dem eine Schub- und Zugstange sich in einem geraden Schaft axial hin und her bewegen kann, um auf diese Weise das Maulteil des Rohrschaftinstruments zu öffnen und zu schließen. Die vor und zurück Bewegung der Schub- und Zugstange wird dabei über einen Gelenkmechanismus an die Branchen des Maulteils übertragen. Die Schubund Zugstange ist als biegefester Stab ausgebildet. Mit einem solchen Prinzip können keine gebogenen Rohrschäfte hergestellt werden.

Auch Rohrschaftinstrumente mit gebogenem Rohrschaft sind im Stand der Technik bereits bekannt. Beispielsweise offenbart die deutsche Patentanmeldung DE 195 20 717 A1 ein Rohrschaftinstrument mit gebogenem Rohrschaft. Bei diesem Rohrschaftinstrument wird ein Schaft verwendet, dessen proximaler Bereich gerade und dessen distaler Bereich gebogen ist. In dem geraden proximalen Bereich wird ein biegesteifer Stab als Schub- und Druckstange verwendet und an dessen distalem Ende wird eine biegeweiche Schub- und Zugstange angebracht. Die biegeweiche Schub- und Zugstange besteht aus einer Stange, in die eine Vielzahl von umlaufenden Nuten eingearbeitet sind, welche den Querschnitt der Stange so weit reduzieren, dass die ursprünglich im Wesentlichen biegesteife Stange biegeweich wird. Zwischen den Nuten sind Abschnitte belassen worden, an denen die Schub- und Zugstange ihren ursprünglichen Durchmesser behalten hat. Diese Abschnitte dienen der korrekten Führung und Stützung der Schub- und Zugstange in dem gebogenen Abschnitt des Schafts. Die Schub- und Zugstange neigt in dem gebogenen Schaftabschnitt dazu, nicht die beabsichtigte Form eines Bogens, wie er durch den gebogenen Bereich des Schafts vorgegeben ist, sondern die Form eines Polygonzugs anzunehmen. Die Anzahl und der Abstand der Stützabschnitte bestimmt dabei die Form des Polygonzugs. Ein derart aufgebauter Rohrschaft kann aber nur mit einem einzigen gebogenen Bereich versehen sein.

In vielen Fällen sind Rohrschaftinstrumente mit standardisierten Biegungen versehen, die für einen bestimmten Einsatzzweck und die an die durchschnittliche Anatomie des Patienten angepasst sind. Wird das Rohrschaftinstrument aber für neue Anwendungen eingesetzt oder weicht die Anatomie des Patienten deutlich von der durchschnittlichen Anatomie ab (z.B. adipöser Patient oder Patient mit Anorexie), passen die vorgefertigten Biegeradien und die Längen der dazwischen angeordneten geraden Schaftabschnitte nicht mehr optimal zu dem Patienten.

Wenn mehrere gebogene Bereiche an einem Rohrschaftinstrument vorgesehen sein sollen oder wenn der Rohrschaft erst unmittelbar vor seinem Einsatz oder sogar erst währenddessen durch den Operateur oder Hilfspersonal gebogen werden soll, muss der Rohrschaft anders aufgebaut sein.

Es ist daher die Aufgabe der vorliegenden Erfindung, einen Rohrschaft für ein Rohrschaftinstrument bereit zu stellen, der an die anatomischen Gegebenheiten eines Patienten angepasst werden kann.

Diese Aufgabe wird gelöst durch einen Rohrschaft für ein Rohrschaftinstrument nach Anspruch 1. Vorteilhafte Ausgestaltungen der Erfindung sind Gegenstand der abhängigen Ansprüche.

Gemäß einer Ausführungsform der vorliegenden Erfindung ist Rohrschaft für ein Rohrschaftinstrument offenbart mit einem hohlen Schaftbauteil, einem in dem hohlen Schaftbauteil angeordneten Betätigungsstab und Funktionselementen, die an den distalen Enden des Schaftbauteils und/oder des Betätigungsstabs angebracht sind. Der Betätigungsstab ist relativ zu dem hohlen Schaftbauteil in axialer Richtung verschiebbar, um dadurch die distalen Abschnitte der Funktionselemente aufeinander zu, aneinander vorbei und/oder voneinander weg zu bewegen, sodass die Funktionsbauteile wie beispielsweise die Branchen von Scheren, Klemmen, Nadelhaltern, etc. ihre jeweilige Funktion ausüben können. Der Betätigungsstab weist wenigstens einen Bereich auf, in dem sich flexible Abschnitte und Stützabschnitte abwechseln und in dem der Betätigungsstab zumindest in einer Querrichtung einen deutlich geringeren Biegewiderstand aufweist als außerhalb dieses mindestens einen Bereichs. Das hohle Schaftbauteil weist zudem mindestens einen Bereich auf, in dem die Wand des Schaftbauteils Aussparungen aufweist und das Schaftbauteil zumindest in einer Querrichtung einen deutlich geringeren Biegewiderstand aufweist als außerhalb dieses mindestens einen Bereichs. Die mindestens einen Bereiche des Betätigungsstabs und des Schaftbauteils überlagern sich in Längsrichtung des Rohrschafts zumindest teilweise, sodass an dem Rohrschaft mindestens ein Bereich ausgebildet wird, in dem der gesamte Rohrschaft gebogen werden kann. Die Biegung des Schaftbauteils findet dabei überwiegend plastisch statt, sodass der Rohrschaft nach dem Biegen in seiner gebogenen Form verbleibt. Die Biegung des Betätigungsstabs kann plastisch und/oder elastisch erfolgen. Der Betätigungsstab wird, wenn er in dem Schaftbauteil hin und her bewegt wird und der Biegeradius des Schaftbauteils über den Weg der Bewegung des Betätigungsstabs zu dem Schaftbauteil nicht absolut gleichbleibend ist, während dieser Bewegung zu einer stärkeren und/oder einer schwächeren Biegung hin verformt. Eine Verbiegung des Betätigungsstabs mit größerem elastischem Anteil führt somit zu einer längeren Lebensdauer des Rohrschafts, sorgt aber gleichzeitig für eine größere Rückstellkraft, die auf das Schaftbauteil einwirkt. Eine Verbiegung des Betätigungsstabs mit einem größeren plastischen Anteil sorgt zwar dafür, dass die Lebensdauer des Betätigungsstabs z.B. aufgrund der Gefahr eines Weißbruchs eine geringere Lebensdauer aufweist, verringert aber gleichzeitig die auf die Schafthülse wirkende Rückstellkraft, wodurch die Schafthülse dünner bzw. filigraner ausgebildet werden kann. Darüber hinaus ist eine reibungsmindernde Schicht an dem wenigstens einen Bereich des Betätigungsstabs vorgesehen, welche die Reibung des Betätigungsstabes an der Innenwand des Schaftbauteils verringert.

Mit einem solchen Aufbau ist es möglich, einen Rohrschaft für ein Rohrschaftinstrument zu schaffen, der vor Ort von Hand in die gewünschte Form gebogen werden kann und somit optimal an den Patienten und das Einsatzgebiet (fachlich wie räumlich) angepasst werden kann. Die Anbringung der Funktionsteile an dem distalen Ende des Schaftbauteils und/oder des Betätigungsstabs erfolgt dabei auf herkömmliche Weise. Ein solcher biegbarer Rohrschaft kann als Einwegbauteil oder als Mehrwegbauteil ausgebildet sein, welches an einem Einweg- oder Mehrweg-Handgriff montierbar ist. Ist der biegbare Rohrschaft als Mehrwegbauteil ausgebildet, muss er zumindest reinigbar und sterilisierbar sein. Bei einem solchen Rohrschaft ist es möglich, dass der Betätigungsstab über deutlich größere Bereiche biegbar ausgebildet ist, als dies für das Schaftbauteil gilt. Allerdings führt eine biegeweiche Ausbildung des Betätigungsstabes zu einer erhöhten Reibung des Betätigungsstabes bzw. der reibungsmindernden Schicht an der Innenwand des Schaftbauteils, da der Betätigungsstab bei einer Schubbelastung zum seitlichen Ausknicken neigt und somit in zusätzliche Anlage an der Innenwand des Schaftbauteils führt. Daher ist es besonders vorteilhaft, den Betätigungsstab nur dort biegeweich auszubilden, wo dies erforderlich ist, nämlich in den Bereichen, in denen das Schaftbauteil biegbar ist. Den Hub des Betätigungsstabs in dem Schaftbauteil muss hierbei natürlich berücksichtigt werden. Die reibungsmindernde Schicht hat zudem zwei Aufgaben. Die erste Aufgabe ist es, die Reibung zwischen dem Betätigungsstab und dem Schaftbauteil zu verringern. Dieses Ziel wird erreicht, indem für diese Schicht ein Material verwendet wird, welches im Zusammenspiel mit dem Schaftbauteil einen geringeren Reibungskoeffizienten aufweist, als es das Material des Betätigungsstabes tut. Üblicherweise wird der Betätigungsstab aus einem Metall hergestellt. Alternativ dazu kann der Betätigungsstab aus einem Kunststoff hergestellt sein und die Reibungsmindernde Schicht wird direkt durch die Oberfläche des Betätigungsstab gebildet. Die zweite Aufgabe der reibungsmindernden Schicht ist es, die Verformungen auszugleichen, die im Biegebereich durch das Biegen der ursprünglich kreisförmigen Querschnitte entsteht. Biegt man ein kreisförmiges Rohr, so verformt sich der Rohrquerschnitt im Biegebereich im Wesentlichen zu einer kreisnahen Ellipse. Würde man für diese Verformungen weder ein Spiel noch eine Ausgleichsschicht vorsehen, wäre der Rohrschaft nach dem Biegen kaum oder gar nicht mehr betätigbar. Sieht man einen Spalt als Spiel zwischen Betätigungsstab und Schaftbauteil vor, geht dies zu Lasten der Genauigkeit bei der Betätigung des Rohrschafts. Aus diesem Grund ist die Verwendung einer Ausgleichsschicht besonders vorteilhaft. Als besonders geeignet zur Verminderung der Reibung zwischen Schaftbauteil und Betätigungsstab haben sich herkömmliche Schrumpfschläuche erwiesen. Diese sind darüber hinaus auch noch sehr gut als Ausgleichsschicht geeignet, da sie hinreichend weich sind, um an den erforderlichen Stellen zwischen dem Schaftbauteil und dem Betätigungsstab - insbesondere dessen Stützabschnitten - zusammen gedrückt zu werden, ohne dabei eine hohe Reibung zwischen den anliegenden Bauteilen zu verursachen. Darüber hinaus sind herkömmliche Schrumpfschläuche besonders einfach an dem Betätigungsstab anzubringen und vergleichsweise günstig in der Herstellung bzw. Beschaffung. Alternativ kann eine reibungsmindernde Schicht auch als Beschichtung auf dem Betätigungsstab oder der Innenwand des Schaftbauteils angebracht werden. Auch eine solche Beschichtung kann die Funktion einer Ausgleichsschicht wahrnehmen.

Gemäß einer vorteilhaften Ausführungsform der vorliegenden Erfindung sind die flexiblen Abschnitte des Betätigungsstabs ausgebildet, indem die maximale Ausdehnung des Querschnitts des Betätigungsstabs in mindestens einer Richtung kleiner als bei einem Stützabschnitt ist.

Mit einer solchen Ausgestaltung verringert sich das Flächenträgheitsmoment und bei einem homogenen Werkstoff automatisch auch das Biegemoment in Bezug auf eine Achse die quer zu der Richtung der verringerten Ausdehnung des Querschnitts verläuft. Prinzipiell können auf diese Weise aber auch mehrere bevorzugte Biegerichtungen geschaffen werden, indem der Betätigungsstab beispielsweise eine rechteckige oder dreieckige Form annimmt. Bei einer quadratischen Form oder der Form eines gleichseitigen Dreiecks ist dabei der Biegewiederstand bzw. die zum Biegen erforderliche Kraft in die durch die Querschnittform des Betätigungsstabs vorgegebenen Richtungen jeweils zueinander identisch. Wenn bei einem Rohrschaft mehr als ein Biegebereich vorgesehen ist, können die Biegungen in jedem Biegebereich in ein und derselben Ebene liegen. Alternativ dazu können die Ebenen, in denen die Biegung in den einzelnen Biegebereichen erfolgt, zueinander verdreht sein. Weiter alternativ ist es auch möglich, dass ein Biegebereich in mehrere Raumrichtungen gleichzeitig gebogen ist, sodass der Rohrschaft an diesem Abschnitt beispielsweise eine spiralform einnimmt.

Gemäß einer weiteren vorteilhaften Ausführungsform der vorliegenden Erfindung sind die flexiblen Abschnitte des Betätigungsstabs ausgebildet, indem Aussparungen in dem Betätigungsstab vorgesehen sind. Auf diese Weise kann der Betätigungsstab zunächst als ein zylindrischer Stab ausgebildet werden, der anschließend mit Aussparungen versehen wird, um im Bereich der Aussparungen die Querschnittfläche des Betätigungsstabs zu verkleinern und somit das Flächenträgheitsmoment und den Biegewiderstand des Betätigungsstabs zu verringern. Bevorzugt sind die Aussparungen als gerade Nuten ausgebildet. Diese können leicht in den Betätigungsstab gefräst oder anderweitig eingebracht werden. Gerade Nuten haben zudem den Vorteil, dass sie eine Richtungsführung für ein Biegen bilden, sodass der Nutzer später eine sehr präzise Biegung des Rohrschafts und damit auch des Betätigungsstabs durchführen kann. Weiter vorzugsweise erstrecken sich die Nuten senkrecht zu der Längsrichtung des Rohrschafts, was eine besonders einfache Herstellung der Nuten ermöglicht.

Gemäß einer anderen vorteilhaften Ausführungsform der vorliegenden Erfindung sind die Aussparungen in dem Betätigungsstab im Wesentlichen auf zwei radial gegenüberliegenden Seiten des Betätigungsstabs angeordnet, um auf diese Weise eine Querrichtung zu definieren, in der die Biegesteifigkeit über den gesamten Bereich geringer als in anderen Querrichtungen ist. Mit dieser Ausgestaltung ist eine bevorzugte Biegeebene für den Betätigungsstab definiert. Dies verhindert, insbesondere im Zusammenspiel mit einem im Folgenden beschriebenen entsprechenden Aufbau des Schaftbauteils, dass der Rohrschaft versehentlich in einer nicht gewünschten Richtung gebogen wird oder ungewünscht in eine dreidimensionale Form gebogen wird.

Gemäß noch einer vorteilhaften Ausführungsform der vorliegenden Erfindung sind die Aussparungen in der Wand des Schaftbauteils im Wesentlichen auf zwei radial gegenüberliegenden Seiten des Schaftbauteils angeordnet, um auf diese Weise eine Querrichtung zu definieren, in der die Biegesteifigkeit über den gesamten Bereich geringer als in anderen Querrichtungen ist. Dieser Aufbau verhindert, dass der Rohrschaft in eine nicht beabsichtigte Richtung gebogen wird oder unerwünscht in eine dreidimensionale Form gebogen wird. Besonders vorteilhaft ist diese Ausgestaltung im Zusammenspiel mit dem vorstehen beschriebenen Aufbau des Betätigungsstabs, aber auch eine entsprechende Ausgestaltung des Schaftbauteils alleine ist in dieser Hinsicht sehr nützlich.

Gemäß noch einer weiteren vorteilhaften Ausführungsform der vorliegenden Erfindung sind die Aussparungen in der Wand des Schaftbauteils gleichmäßig entlang des Radius des Schaftbauteils verteilt angeordnet, sodass die Biegesteifigkeit des Schaftbauteils in alle Querrichtungen im Wesentlichen gleich ist. Mit dieser Ausgestaltung gibt man dem Nutzer die Möglichkeit, den Rohrschaft in eine beliebige gewünschte Form zu biegen. Dabei muss der Nutzer allerdings auf die Führung in einer bevorzugten Biegeebene verzichten. Diese Formulierung soll auch eine sehr breite umlaufende Nut in der Außenwand des Schaftbauteils umfassen, die als Ergebnis einer Überlagerung vieler einzelner Aussparungen zu sehen ist.

Gemäß noch einer anderen vorteilhaften Ausführungsform der vorliegenden Erfindung sind die Aussparungen in der Wand des Schaftbauteils zumindest teilweise als Durchgangsöffnungen ausgebildet. Die Aussparungen, die zumeist als Nuten ausgebildet sind, können alternativ dazu an ihrem Boden verschlossen sein. In diesem Fall kann verhindert werden, dass Fremdstoffe in das Innere des Schaftbauteils eindringen und eventuell das Handling des Rohrschafts beeinträchtigen. Der Boden einer jeden solchen Nut kann auch so dünn ausgebildet sein, dass er unter Umständen während des Biegens des Rohrschafts bricht. Die Aussparungen sind vorzugsweise als gerade Nuten ausgebildet, die sich weiter vorzugsweise senkrecht zu der Längsrichtung des Rohrschafts erstrecken. Dies ermöglicht eine besonders einfache Herstellung der Aussparungen in dem Schaftbauteil.

Gemäß wieder einer anderen vorteilhaften Ausführungsform der vorliegenden Erfindung ist an dem Betätigungsstab und/oder an dem Schaftbauteil eine Mehrzahl von Biegebereichen ausgebildet. Zwischen zwei benachbarten Biegebereichen ist dabei ein Bereich vorgesehen, an dem der Betätigungsstab und/oder das Schaftbauteil im Wesentlichen biegesteif ist. Solch ein Aufbau ist insofern vorteilhaft, als dass bei einem Rohrschaft mit mehr als einem Biegebereich die Reibung des Betätigungsstabs bzw. der reibungsmindernden Schicht an oder auf dem Betätigungsstab an der Innenwand des Schaftbauteils verringert wird. Ist der Betätigungsstab auch in den geraden Bereichen des Rohrschafts biegeweich ausgebildet, neigt er dazu, seitlich auszuknicken und sich an vielen punkten an der Innenwand des Schaftbauteils anzulehnen, was zu einer erhöhten Reibung führt. Mit einer im Wesentlichen biegesteifen Ausbildung der geraden Bereiche des Betätigungsstabes wird dieser Effekt im Wesentlichen vollständig verhindert und somit die Reibung zwischen Betätigungsstab bzw. reibungsmindernder Schicht und Schaftbauteil so gering wie möglich gehalten.

Gemäß noch einer anderen vorteilhaften Ausführungsform der vorliegenden Erfindung erstreckt sich der mindestens eine Bereich, in dem der Betätigungsstab eine geringere Biegesteifigkeit aufweist, in der Längsrichtung des Rohrschafts zumindest über im Wesentlichen den gesamten mindestens einen Bereich, in dem die Biegesteifigkeit des Schaftbauteils verringert ist. Das Schaftbauteil als äußeres Bauteil weist eine größere Ausdehnung des Querschnitts auf als der Betätigungsstab. Aus diesem Grund weist das Schaftbauteil auch in den Bereichen, die mit Aussparungen versehen sind und daher eine verringerte Biegesteifigkeit aufweisen, ein größeres Flächenträgheitsmoment um die jeweilige Biegeachse auf als der Betätigungsstab in einem Bereich, in dem er eine geringere Biegesteifigkeit aufweist. Dies gilt insbesondere auch, da Betätigungsstab und Schaftbauteil üblicherweise so dimensioniert sind, dass sie die erforderlichen Kräfte übertragen können, ohne einen übermäßig großen Querschnitt aufzuweisen, da dies zu Lasten der erwünschten Kompaktheit des Rohrschafts ginge. Zudem sind der Betätigungsstab und das Schaftbauteil üblicherweise aus demselben Material gefertigt. Daher weist das Schaftbauteil in den genannten Bereichen eine normalerweise deutlich höhere Biegesteifigkeit auf als der Betätigungsstab. Bei einemBiegen des Rohrschafts durch den Nutzer bestimmt demnach vor allem das Schaftbauteil die aufzuwendende Kraft. Damit ein Nutzer nicht versehentlich das Schaftbauteil in einem Bereich biegt, in dem zwar das Schaftbauteil für ein Biegen vorgesehen ist, der Betätigungsstab aber nicht, was dann dazu führt, dass der gebogene Rohrschaft nicht mehr funktionsfähig ist, ist der Betätigungsstab zumindest überall dort, wo das Schaftbauteil für ein Biegen vorbereitet ist, ebenfalls für ein Biegen vorbereitet, d.h. er weist dort eine verringerte Biegesteifigkeit auf.

Gemäß wieder einer weiteren vorteilhaften Ausführungsform der vorliegenden Erfindung stimmt die durch die Aussparungen des Betätigungsstabs definierte Querrichtung mit der durch die Aussparungen in dem Schaftbauteil definierte Querrichtung im Wesentlichen überein. Auf diese Weise ist sicher gestellt, dass der Nutzer, der den Rohrschaft biegt, eine klare Führung der Biegung in der so eingerichteten Biegeebene erfährt. Außerdem wird auf diese Weise sicher gestellt, dass die in dem Rohrschaft herrschenden Kräfte zwischen Schaftbauteil und Betätigungsstab minimal sind. Wären diese Querrichtungen zueinander deutlich verdreht, könnte dies zu einem Ausbeulen des Betätigungsstabs in dem Schaftbauteil führen, was unnötige Reibung zwischen diesen Bauteilen verursacht.

Gemäß einer anderen vorteilhaften Ausführungsform der vorliegenden Erfindung sind der Betätigungsstab und die Schafthülse daran angepasst, in dem mindestens einen Bereich einen in Längsrichtung des Rohrschafts veränderlichen Biegeradius zu ermöglichen. Dies kann beispielsweise durch unterschiedliche große oder viele Aussparungen vorgegeben werden, die in dem Schaftbauteil und/oder dem Betätigungsstab vorgesehen sind. Die Aussparungen können auch, wenn sie als Nuten ausgebildet sind, als dreieckige Nuten ausgebildet sein, deren Flanken unterschiedlich steil angeordnet sind. Eine Biegung ist dann genau so weit möglich, bis sich die Flanken einer Nut berühren. Auf diese Weise kann jedem Bereich in Längsrichtung des Rohrschafte eine maximal erzielbare Biegung vorgegeben werden.

Gemäß noch einer anderen vorteilhaften Ausführungsform der vorliegenden Erfindung ist eine optische und/oder haptische Kennzeichnung mindestens eines Biegebereiches vorgesehen, welche den mindestens einen Bereich des Rohrschafts in dessen Längsrichtung kennzeichnet, an dem sich ein Bereich des Betätigungsstabs mit geringem Biegewiderstand und ein Bereich des Schaftbauteils mit geringem Biegewiederstand befinden. Auf diese Weise kann dem Nutzer einfach angezeigt werden, in welchen Bereichen eine Biegung vorgenommen werden kann, ohne die Funktionalität des Rohrschafts zu beeinträchtigen. Gleichzeitig kann dem Nutzer eine Biegeebene angezeigt werden, in der die Biegung bestenfalls vorgenommen werden soll, oder es kann angezeigt werden, dass in einem gewissen Bereich eine dreidimensionale Biegung möglich ist.

Gemäß noch einer weiteren vorteilhaften Ausführungsform der vorliegenden Erfindung weist der Rohrschaft eine Verkleidung auf, die das Schaftbauteil bedeckt, und die Kennzeichnung des mindestens einen Biegebereichs an der Verkleidung vorgesehen ist. Eine Verkleidung ist vorteilhaft, um beispielsweise die Reibung eines Rohrschafts in einem Trokar zu minimieren. Zudem dient eine solche Verkleidung auch als Schutz gegen ein Eindringen von Verschmutzungen in den Rohrschaft, insbesondere wenn die in dem Schaftbauteil vorgesehenen Aussparungen als Durchgangslöcher ausgebildet sind oder bei einem Biegevorgang geöffnet werden. Die Verkleidung ist vorzugsweise so elastisch, dass sie nicht beschädigt wird, wenn der Rohrschaft gebogen wird.

Gemäß wieder einer anderen vorteilhaften Ausführungsform der vorliegenden Erfindung kennzeichnet die Kennzeichnung das distale und proximale Ende des mindestens einen Biegebereichs und kennzeichnet bevorzugt auch den Mittelpunkt des mindestens einen Biegebereichs. Mit einer derartigen Ausbildung kann der Nutzer den Rohrschaft zum Biegen an Bereichen außerhalb des Biegebereichs greifen, an denen der Rohrschaft stabiler ist, sodass der Rohrschaft nicht durch das Greifen versehentlich beschädigt wird, beispielsweise indem eine durch die Aussparungen geschwächte Wand des Schaftbauteils nach innen in einen Bereich zwischen zwei Stützabschnitte verformt wird, was die Funktionsfähigkeit des Rohrschafts beeinträchtigen würde. Ist zudem der Mittelpunkt des Biegebereichs gekennzeichnet, weiß der Nutzer, an welcher Stelle bzw. in welchem Bereich die maximale Krümmung des Rohrschafts bestenfalls geschaffen werden sollte.

Gemäß wieder einer anderen vorteilhaften Ausführungsform der vorliegenden Erfindung gibt die Kennzeichnung einen Hinweis darauf, welcher Biegeradius und/oder welche Biegerichtung an welcher Stelle möglich ist, bevorzugt durch Verwendung verschiedener Farben oder unterschiedlich großer oder nah beieinander angeordneter Symbole oder Linien. Eine Biegung, die manuell durch den Nutzer am Ort des Einsatzes des Rohrschafts vorgenommen wird, wird weniger zu einem gebogenen Rohrschaft mit einem kreisbogenförmigen Rohrschaftabschnitt führen, sondern der gebogene Abschnitt des Rohrschafts wird eher den Verlauf einer Klothoide annehmen, also eines Bogens mit veränderlichem Krümmungsradius. Damit der Nutzer in den Randbereichen eines Biegebereichs eines solchen Rohrschafts keine Überbiegung des Rohrschafts vornimmt, kann ihm auf diese Weise ein Hinweis darauf gegeben werden, welcher Krümmungsradius erreicht werden kann, ohne die Funktionalität des Rohrschafts in unerwünschter Weise zu beeinträchtigen.

Gemäß noch einer weiteren vorteilhaften Ausführungsform der vorliegenden Erfindung sind die Abstände der Aussparungen in der Wand des Schaftbauteils und die Abstände der Stützabschnitte des Betätigungsstabs sowie die Lage von Schaftbauteil und Betätigungsstab so aufeinander abgestimmt, dass kein Stützabschnitt des Betätigungsstabs in eine Aussparung in der Wand des Schaftbauteils eintreten kann. Andernfalls kann es passieren, dass in einer gewissen Stellung des Betätigungsstabs zu dem Schaftbauteil bei einer Biegung des Rohrschafts ein Stützabschnitt in eine in dem Schaftbauteil vorgesehene Nut eintritt und bei einer Biegung diese Nut verengt wird, wobei der Stützabschnitt des Betätigungsstabs in dieser Nut verklemmt wird. Ein solcher Rohrschaft wäre funktionsuntüchtig. Um dieses Ziel zu erreichen kann beispielsweise der Abstand zwischen zwei benachbarten Nuten in dem Schaftbauteil deutlich größer gewählt werden als ein Stützabschnitt breit ist und der Hub der beiden Bauteile zueinander lang ist. Alternativ kann ein Stützabschnitt breiter ausgebildet werden, als eine Nut des Schaftbauteils sich selbst auf der Außenseite der Biegung aufweiten kann.

Weitere Vorteile und Merkmale der Erfindung sind dem Fachmann aus den beigefügten Figuren und der detaillierten Beschreibung der Ausführungsbeispiele ersichtlich.
- Fig. 1: zeigt eine erste Ausführungsform eines Schaftbauteils, wobei Fig. 1A eine isometrische Ansicht, Fig. 1B eine isometrische Ansicht des distalen Endes, Fig. 1C eine perspektivische Ansicht des distalen Endes im gebogenen Zustand und Fig. 1D eine Draufsicht auf das distale Ende des Schaftbauteils zeigt;
- Fig. 2: zeigt eine zweite Ausführungsform eines Schaftbauteils, wobei Fig. 2A eine isometrische Ansicht eines distalen Endes und Fig. 2B eine Draufsicht auf das distale Ende des Schaftbauteils zeigt;
- Fig. 3: zeigt eine dritte Ausführungsform eines Schaftbauteils, wobei Fig. 3A eine isometrische Ansicht eines distalen Endes und Fig. 3B eine Draufsicht auf das distale Ende des Schaftbauteils zeigt;
- Fig. 4: zeigt eine erste Ausführungsform eines Betätigungsstabs, wobei Fig. 4A eine isometrische Ansicht und Fig. 4B eine perspektivische Ansicht des distalen Endes des Betätigungsstabs zeigt; und
- Fig. 5: zeigt eine zweite Ausführungsform eines Betätigungsstabs, wobei Fig. 5A eine perspektivische Ansicht, Fig. 5B eine perspektivische Ansicht im gebogenen Zustand und Fig. 5C einen Ausschnitt der Fig. 5A zeigt.

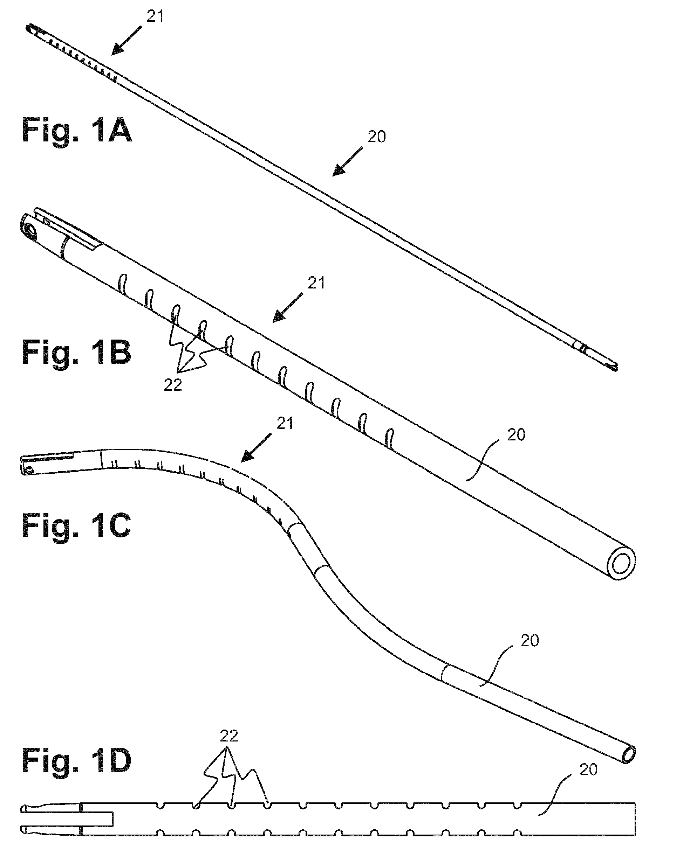

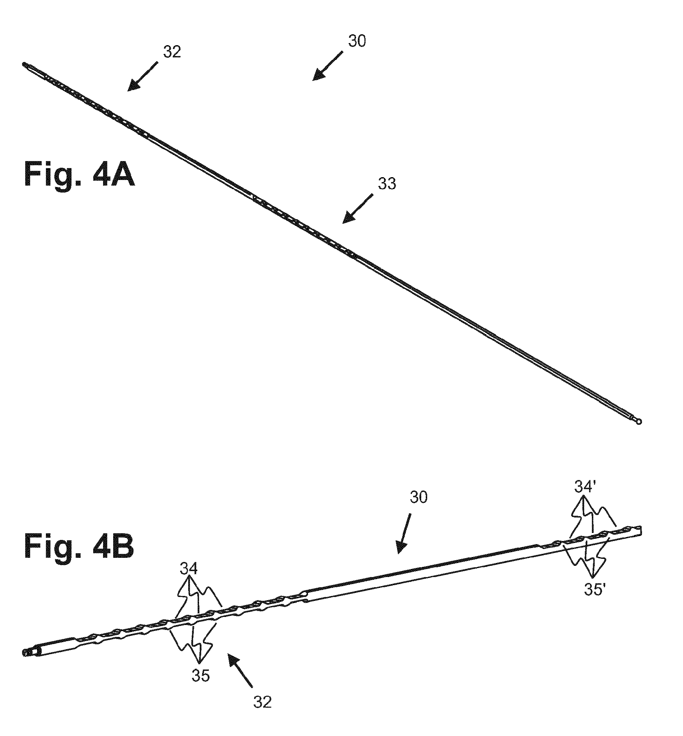

Im Folgenden sind die Ausführungsbeispiele der vorliegenden Erfindung unter Bezugnahme auf die Figuren im Detail beschrieben. Ein erstes Ausführungsbeispiel ist unter Bezugnahme auf die Fig. 1 und 4 im Detail beschrieben.

In der Fig. 1A ist ein Rohrschaft für ein Rohrschaftinstrument gezeigt mit einem hohlen Schaftbauteil 20, einem in dem hohlen Schaftbauteil 20 angeordneten Betätigungsstab 30, und Funktionselementen (nicht gezeigt), die an den distalen Enden des Schaftbauteils 20 und des Betätigungsstabs 30 angebracht sind.

Am proximalen Ende des Rohrschafts ist eine Kopplung ausgebildet, mit welcher der Rohrschaft an einem Griffbauteil lösbar montierbar ist. Der Rohrschaft ist bei diesem Ausführungsbeispiel als Einwegartikel konzipiert, der an einem wiederverwendbaren Griffteil (nicht gezeigt) montierbar ist. Die Schafthülse 20 weist zur Kopplung Kugelaufnahmen auf, in die Kugeln als Sicherungselemente eintauchen, um so die Schafthülse 20 an dem Griffteil zu halten. Der Betätigungsstab 30 weist an seinem proximalen Ende eine Kupplungskugel auf, die mit einer entsprechenden Aufnahme am Griffteil lösbar koppelbar ist, um eine Betätigung des Betätigungselements des Griffteils auf den Betätigungsstab 30 zu übertragen und so eine Funktion der Funktionsteile zu gewährleisten.

Der Betätigungsstab 30 ist relativ zu dem hohlen Schaftbauteil 20 in axialer Richtung verschiebbar ist, um dadurch die distalen Abschnitte der Funktionselemente aufeinander zu, aneinander vorbei und voneinander weg zu bewegen. Bei diesem Ausführungsbeispiel sind die Funktionselemente Branchen einer Schere. Der Betätigungsstab 30 weist zwei Bereiche 32, 33 auf, in dem sich flexible Abschnitte 34, 34' und Stützabschnitte 35, 35' abwechseln. Wie dies in der Fig. 4B gezeigt ist, sind die beiden Bereiche 32, 33 unterschiedlich aufgebaut. Während in dem Bereich 32 die flexiblen Stützabschnitte 34 ausgebildet sind, indem der Betätigungsstab 30 von gegenüberliegenden Seiten her tailliert ist bzw. mit Nuten versehen ist und die Stützabschnitte 35 zu zwei zur Achse gegenüberliegenden Seiten gegenüber den flexiblen Abschnitten 34 vorstehen, sind in dem Bereich 33 die flexiblen Abschnitte 34' nur von einer Seite her tailliert bzw. mit Nuten versehen, sodass die Stützabschnitte 35' auch nur zu einer Seite gegenüber den flexiblen Abschnitten 34' hervorstehen. Auf diese Weise weist der Betätigungsstab 30 in beiden Bereichen 32, 33 zumindest in einer Querrichtung einen deutlich geringeren Biegewiderstand auf als außerhalb dieser Bereiche 32, 33.

Das hohle Schaftbauteil 20 weist ebenfalls zwei Bereiche auf, in dem die Wand des Schaftbauteils 20 Aussparungen 21 aufweist, wobei in den Figuren nur der distale Bereich 21 gezeigt ist. Das Schaftbauteil 20 entsprechend diesem Ausführungsbeispiel weist in diesem Bereich in einer Querrichtung einen deutlich geringeren Biegewiderstand auf als außerhalb dieses mindestens einen Bereichs 21, wobei sich die beiden Bereiche 21, 32, 33 des Betätigungsstabs 20 und des Schaftbauteils 30 in Längsrichtung des Rohrschafts überlagern.

Eine reibungsmindernde Schicht, die in den Figuren ebenfalls nicht gezeigt ist, ist an den beiden Bereichen 21 des Betätigungsstabs 20 vorgesehen, um die Reibung des Betätigungsstabes 20 an der Innenwand des Schaftbauteils 30 zu verringern. Bei diesem Ausführungsbeispiel ist die reibungsmindernde Schicht durch einen Schrumpfschlauch gebildet.

Wie dies vorstehend angedeutet ist, sind die flexiblen Abschnitte 34, 34' des Betätigungsstabs 30 ausgebildet, indem die maximale Ausdehnung des Querschnitts des Betätigungsstabs 30 in einer Richtung kleiner als bei einem Stützabschnitt 35, 35' ist. Die flexiblen Abschnitte 34, 34' sind durch gerade Nuten ausgebildet, die sich senkrecht zu der Längsrichtung des Rohrschafts erstrecken. Die Aussparungen 22 in der Wand des Schaftbauteils 20 sind auf zwei radial gegenüberliegenden Seiten des Schaftbauteils 20 angeordnet und definieren auf diese Weise eine Querrichtung, in der die Biegesteifigkeit über den gesamten Bereich 21 geringer als in anderen Querrichtungen ist. Diese Aussparungen 22 sind ebenfalls als Nuten ausgebildet, die in das Schaftbauteil gefräst wurden und senkrecht zu der Längsrichtung des ungebogenen Schaftbauteils verlaufen. Diese Nuten 22 öffnen sich nicht zum Inneren des Schaftbauteils, auch nicht im gebogenen zustand, der in Fig. 1C gezeigt ist.

Zwischen den beiden Bereichen 32, 33 des Betätigungsstabs 30 und den beiden Bereichen 21 des Schaftbauteils 20 (von denen nur einer in den Fig. gezeigt ist) sind der Betätigungsstab 30 und das Schaftbauteil 20 im Wesentlichen biegesteif ausgebildet. Der Begriff biegesteif bezieht sich hier auf die bestimmungsgemäß zu erwartenden Kräfte und Momente, welche auf den Rohrschaft einwirken, wobei hier auch die Biegung des Rohrschafts an den vorhergesehenen Bereichen durch den Nutzer zum bestimmungsgemäßen Gebrauch des Rohrschafts gehört.

Die Bereiche 32, 33 des Betätigungsstabes 30, welche für eine Biegung vorgesehen sind, und die Bereiche 21 des Schaftbauteils 20, welche ebenfalls für eine Biegung vorgesehen sind, überdecken sich im Wesentlichen vollständig, wobei die Bereiche 32, 33 an dem Betätigungsstab 30 ein wenig größer sind, da der Hub des Betätigungsstabs 30 gegenüber dem Schaftbauteil 20 berücksichtigt werden muss. Dies bedeutet, dass die Bereiche 32, 33 um im Wesentlichen die Länge des Hubs des Rohrschafts größer als die Bereiche 21 des Schaftbauteils sind. Der Betätigungsstab 30 ist in dem Schaftbauteil 20 so angeordnet, dass die durch die Aussparungen des Betätigungsstabs definierte Querrichtung mit der durch die Aussparungen in dem Schaftbauteil definierte Querrichtung im Wesentlichen überein stimmt.

In der fig. 1C ist das distale Ende des Schaftbauteils 20 im gebogenen Zustand gezeigt. Eine entsprechende Biegung kann auch hergestellt werden, wenn der Betätigungsstab 30 in dem Schaftbauteil 20 angeordnet ist.

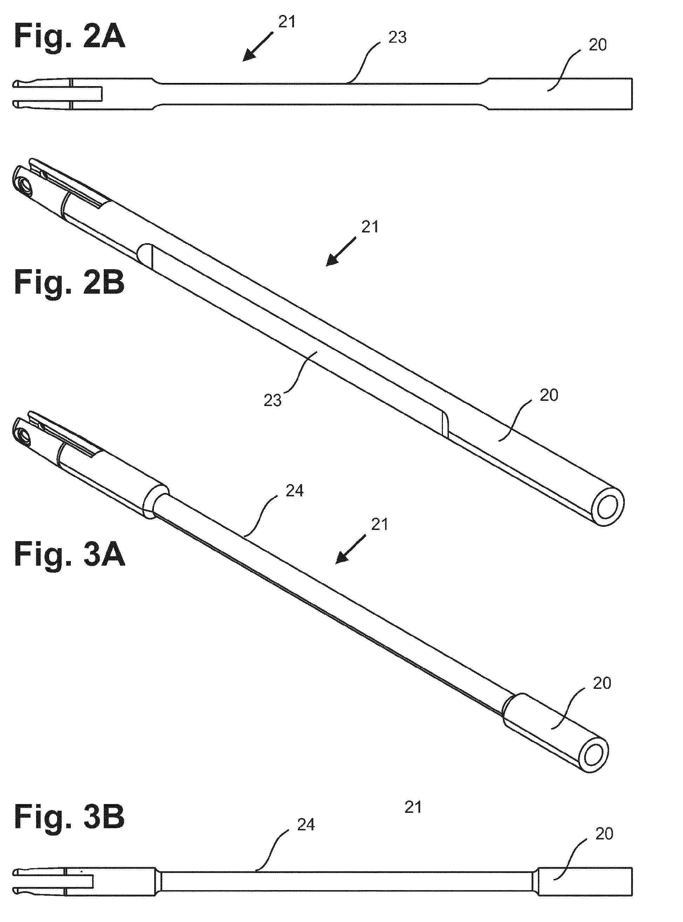

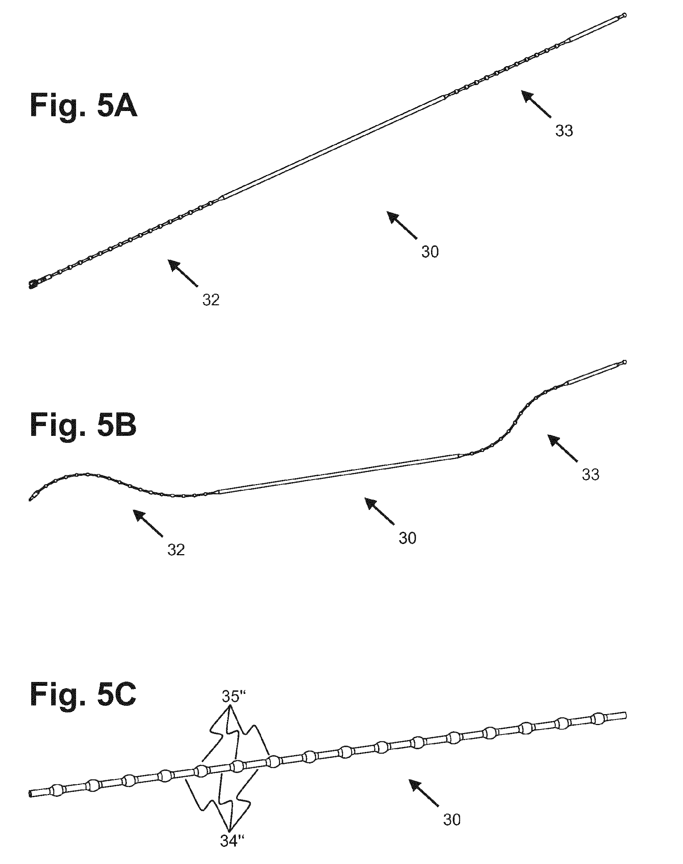

Ein zweites Ausführungsbeispiel der vorliegenden Erfindung ist unter Bezugnahme auf die Fig. 2 und 5 im Detail beschrieben. Im Folgenden sind nur die Unterscheide zu dem ersten Ausführungsbeispiel beschrieben.

Das Schaftbauteil 20 weist bei diesem Ausführungsbeispiel ebenfalls zwei Bereiche auf, in denen er durch einen Nutzer gebogen werden kann. In den Fig. 2A und 2B ist aber wiederum nur der distale Bereich 21 der beiden Bereiche gezeigt. Im Unterschied zu dem ersten Ausführungsbeispiel ist bei diesem Ausführungsbeispiel das Schaftbauteil 20 an zwei gegenüberliegenden Seiten (bezogen auf die Langsachse des Schaftbauteils) mit je einer breiten Aussparung 23 versehen, die in Form einer senkrecht zu der Längsachse verlaufenden Nut 23 ausgebildet ist.

Der Betätigungsstab 20 dieses Ausführungsbeispiels unterscheidet sich von dem ersten Ausführungsbeispiel dahingehend, dass die beiden zum Biegen vorgesehenen Bereiche in einer Art perlenkettenform ausgebildet sind. Dies bedeutet, dass die flexiblen Abschnitte 34", welche in der Fig. 5C gezeigt sind, einen Kreisquerschnitt aufweisen und somit in alle Richtungen quer zur Längsachse des Betätigungsstabs 30 einen identischen Biegewiderstand aufweisen. Die Stützabschnitte 35" sind im Wesentlichen kugelförmig ausgebildet, wobei ein ausgerundeter Übergang zwischen flexiblen Abschnitten 34" und Stützabschnitten 35" ausgebildet ist. Die Biegeebene, in der der Rohrschaft bevorzugt gebogen werden kann, ist bei diesem Ausführungsbeispiel ausschließlich durch das Schaftbauteil 20 vorgegeben. Dies hat den Vorteil, dass beim Zusammenbau von Betätigungsstab 20, reibungsmindernder Schicht und Schaftbauteil 30 nicht auf die Drehung des Betätigungsstabs 30 zu dem Schaftbauteil 20 geachtet werden muss, was die Montage deutlich vereinfacht und beschleunigt. Wie dies in der Fig. 4A zudem angedeutet ist, ist das Kulissenelement zur Steuerung der Funktionsteile mittels einer Drehhülse an dem distalen Ende des Betätigungsstabs 30 montiert, sodass auch aus diesem Grund keine rotatorische Ausrichtung des Betätigungsstabs 30 zu dem Schaftbauteil 20 zu erfolgen hat.

Ein drittes Ausführungsbeispiel der vorliegenden Erfindung ist im Folgenden unter Bezugnahme auf die Fig. 3 und 5 im Detail beschrieben. Hier sind jeweils nur die Unterschiede zu dem zweiten Ausführungsbeispiel beschrieben.

Im Gegensatz zu dem zweiten Ausführungsbeispiel weist das Schaftbauteil 20 des dritten Ausführungsbeispiels zwei zum Biegen bestimmte Bereiche auf, die keine Biegeebene vorgeben. Auch in der Fig. 3 ist nur der distale Bereich 21 der beiden zum Biegen vorgesehenen Bereiche gezeigt. Genauer gesagt sind die Aussparungen 24 in der Wand des Schaftbauteils 20 gleichmäßig entlang des Radius des Schaftbauteils 20 verteilt angeordnet, sodass die Biegesteifigkeit des Schaftbauteils 20 in alle Querrichtungen im Wesentlichen gleich ist. Dies bedeutet, dass der Rohrschaft des dritten Ausführungsbeispiels für eine dreidimensionale Biegung geeignet ist, dem Nutzer dafür aber keine Richtungsführung für das Biegen bietet. Ist bei einem solchen Aufbau ein dreidimensionales Biegen trotzdem nicht erwünscht, so kann eine bevorzugte oder beabsichtigte Biegeebene für beide Bereiche durch eine entsprechende Kennzeichnung auf dem Schaftbauteil 20 oder einer Verkleidung desselben angegeben sein.

Um den Nutzer das Biegen des Rohrschafts zu erleichtern und die Gefahr zu verringern, dass der Rohrschaft beim Biegen versehentlich geknickt wird, kann eine Schablone verwendet werden. Solche eine Schablone kann einen festen Radius aufweisen und es können verschiedene Schablonen mit verschiedenen Radien verwendet werden. Im Stand der Technik sind Kurvenlineale bekannt, die über mehrere solche veränderlichen Radien verfügen. Alternativ dazu kann eine Schablone verwendet werden, die verschiedene Radien aufweist oder mindestens einen Bogen mit veränderlichen Radius. Für Formen von gebogenen Rohrschäften, die häufiger Einsatz finden, können auch Schablonen verwendet werden, die feste Radien für mehrere Biegebereiche des Rohrschafts aufweisen. Bei all den vorstehend beschriebenen Biegeschablonen ist es vorteilhaft, wenn die Biegekante eine Nut aufweist, die einen Teil des Schaftbauteils 20 aufnehmen kann, sodass das Schaftbauteil 20 durch diese Nut gehalten ist und nicht von dem Biegeradius abrutscht. Vorteilhafter Weise weist diese Nut einen halbkreisförmigen Querschnitt auf und/oder sie besitzt im Wesentlichen denselben Durchmesser wie das Schaftbauteil 20.

Der Fachmann kann die beschriebenen Merkmale des Rohrschafts aus verschiedenen Ausführungsbeispielen in geeigneter Weise beliebig kombinieren.

## Patentansprüche

1. Rohrschaft für ein Rohrschaftinstrument mit
einem hohlen Schaftbauteil (20),
einem in dem hohlen Schaftbauteil (20) angeordneten Betätigungsstab (30), und
Funktionselementen, die an den distalen Enden des Schaftbauteils (20) und/oder des Betätigungsstabs (30) angebracht sind, wobei der Betätigungsstab (30) relativ zu dem hohlen Schaftbauteil (20) in axialer Richtung verschiebbar ist, um dadurch die distalen Abschnitte der Funktionselemente aufeinander zu, aneinander vorbei und/oder voneinander weg zu bewegen,
**dadurch gekennzeichnet, dass**
der Betätigungsstab (30) wenigstens einen Bereich (32, 33) aufweist, in dem sich flexible Abschnitte (34, 34', 34") und Stützabschnitte 35, 35', 35" abwechseln und in dem der Betätigungsstab (30) zumindest in einer Querrichtung einen deutlich geringeren Biegewiderstand aufweist als außerhalb dieses mindestens einen Bereichs (32, 33), und
das hohle Schaftbauteil (20) mindestens einen Bereich (21) aufweist, in dem die Wand des Schaftbauteils (20) Aussparungen (22) aufweist und das Schaftbauteil (20) zumindest in einer Querrichtung einen deutlich geringeren Biegewiderstand aufweist als außerhalb dieses mindestens einen Bereichs (21), wobei sich die mindestens einen Bereiche (21, 32, 33) des Betätigungsstabs (20) und des Schaftbauteils in Längsrichtung des Rohrschafts zumindest teilweise überlagern, und
eine reibungsmindernde Schicht an dem wenigstens einen Bereich (32, 33) des Betätigungsstabs vorgesehen ist, welche die Reibung des Betätigungsstabes (30) an der Innenwand des Schaftbauteils (20) verringert, wobei die reibungsmindernde Schicht bevorzugt durch einen Schrumpfschlauch gebildet ist.

2. Rohrschaft für ein Rohrschaftinstrument nach Anspruch 1, wobei
die flexiblen Abschnitte (32, 33) des Betätigungsstabs (30) ausgebildet sind, indem die maximale Ausdehnung des Querschnitts des Betätigungsstabs (30) in mindestens einer Richtung kleiner als bei einem Stützabschnitt (35, 35', 35") ist.

3. Rohrschaft für ein Rohrschaftinstrument gemäß Anspruch 1 oder 2, wobei
die flexiblen Abschnitte (35', 35") des Betätigungsstabs (30) ausgebildet sind, indem Aussparungen in dem Betätigungsstab (30) vorgesehen sind, welche bevorzugt als gerade Nuten ausgebildet sind, die sich weiter bevorzugt senkrecht zu der Längsrichtung des Rohrschafts erstrecken.

4. Rohrschaft für ein Rohrschaftinstrument gemäß Anspruch 3, wobei
die Aussparungen in dem Betätigungsstab (30) im Wesentlichen auf zwei radial gegenüberliegenden Seiten des Betätigungsstabs (30) angeordnet sind, um auf diese Weise eine Querrichtung zu definieren, in der die Biegesteifigkeit über den gesamten Bereich geringer als in anderen Querrichtungen ist.

5. Rohrschaft für ein Rohrschaftinstrument gemäß einem der vorangehenden Ansprüche, wobei
die Aussparungen (22) in der Wand des Schaftbauteils (20) im Wesentlichen auf zwei radial gegenüberliegenden Seiten des Schaftbauteils (20) angeordnet sind, um auf diese Weise eine Querrichtung zu definieren, in der die Biegesteifigkeit über den gesamten Bereich geringer als in anderen Querrichtungen ist.

6. Rohrschaft für ein Rohrschaftinstrument gemäß einem der vorangehenden Ansprüche, wobei
die Aussparungen (22) in der Wand des Schaftbauteils (20) gleichmäßig entlang des Radius des Schaftbauteils (20) verteilt angeordnet sind, sodass die Biegesteifigkeit des Schaftbauteils (20) in alle Querrichtungen im Wesentlichen gleich ist.

7. Rohrschaft für ein Rohrschaftinstrument gemäß einem der vorangehenden Ansprüche, wobei
die Aussparungen (22) in der Wand des Schaftbauteils (20) zumindest teilweise als Durchgangsöffnungen ausgebildet sind und/oder als gerade Nuten ausgebildet sind, die sich bevorzugt senkrecht zu der Längsrichtung des Rohrschafts erstrecken.

8. Rohrschaft für ein Rohrschaftinstrument gemäß einem der vorangehenden Ansprüche, wobei
an dem Betätigungsstab (30) und/oder an dem Schaftbauteil (20) eine Mehrzahl von Biegebereichen (21, 32, 33) ausgebildet ist und wobei zwischen zwei benachbarten Biegebereichen (21, 32, 33) ein Bereich vorgesehen ist, an dem der Betätigungsstab (30) und/oder das Schaftbauteil (20) im Wesentlichen biegesteif ist.

9. Rohrschaft für ein Rohrschaftinstrument gemäß einem der vorangehenden Ansprüche, wobei
der mindestens eine Bereich, in dem der Betätigungsstab (30) eine geringere Biegesteifigkeit aufweist, sich in der Längsrichtung des Rohrschafts zumindest über im Wesentlichen den gesamten mindestens einen Bereich (21) erstreckt, in dem die Biegesteifigkeit des Schaftbauteils (20) verringert ist.

10. Rohrschaft für ein Rohrschaftinstrument gemäß einem der vorangehenden Ansprüche, wobei
die durch die Aussparungen des Betätigungsstabs (30) definierte Querrichtung mit der durch die Aussparungen (22) in dem Schaftbauteil (20) definierte Querrichtung im Wesentlichen überein stimmt.

11. Rohrschaft für ein Rohrschaftinstrument gemäß einem der vorangehenden Ansprüche, wobei
der Betätigungsstab (30) und das Schaftbauteil (20) daran angepasst sind, in dem mindestens einen Bereich einen in Längsrichtung des Rohrschafts veränderlichen Biegeradius zu ermöglichen.

12. Rohrschaft für ein Rohrschaftinstrument gemäß einem der vorangehenden Ansprüche, wobei
eine optische und/oder haptische Kennzeichnung mindestens eines Biegebereiches (21, 32, 33) vorgesehen ist, welche den mindestens einen Bereich des Rohrschafts in dessen Längsrichtung kennzeichnet, an dem sich ein Bereich des Betätigungsstabs (30) mit geringem Biegewiderstand und ein Bereich des Schaftbauteils (20) mit geringem Biegewiederstand befinden, wobei
der Rohrschaft vorzugsweise eine Verkleidung aufweist, die das Schaftbauteil (20) bedeckt, und die Kennzeichnung des mindestens einen Biegebereichs an der Verkleidung vorgesehen ist.

13. Rohrschaft für ein Rohrschaftinstrument gemäß Anspruch 12, wobei
die Kennzeichnung das distale und proximale Ende des mindestens einen Biegebereichs (21, 32, 33) kennzeichnet und bevorzugt auch den Mittelpunkt des mindestens einen Biegebereichs (21, 32, 33) kennzeichnet, wobei
die Kennzeichnung vorzugsweise einen Hinweis darauf gibt, welcher Biegeradius und/oder welche Biegerichtung an welcher Stelle möglich ist, bevorzugt durch Verwendung verschiedener Farben oder unterschiedlich großer oder nah beieinander angeordneter Symbole oder Linien.

14. Rohrschaft für ein Rohrschaftinstrument gemäß einem der vorangehenden Ansprüche, wobei
die Abstände der Aussparungen (22) in der Wand des Schaftbauteils (20) und die Abstände der Stützabschnitte (35, 35', 35") des Betätigungsstabs (30) sowie die Lage von Schaftbauteil (20) und Betätigungsstab (30) in axialer Richtung so aufeinander abgestimmt sind, dass kein Stützabschnitt 35, 35', 35" des Betätigungsstabs (30) in eine Aussparung (22) in der Wand des Schaftbauteils (20) eintreten kann.

15. Verwendung einer Biegeschablone zum Biegen eines Rohrschafts für ein Rohrschaftinstrument.
